(19) Europäisches Patentamt
European Patent Office
Office européen des brevets

(11) Numéro de publication : **0 465 342 B1**

(12) **FASCICULE DE BREVET EUROPEEN**

(45) Date de publication du fascicule du brevet : **29.09.93 Bulletin 93/39**

(21) Numéro de dépôt : **91401804.9**

(22) Date de dépôt : **02.07.91**

(51) Int. Cl.$^5$ : **A61K 7/09,** C07D 211/88, C07D 209/48, C07D 207/44, C07C 323/40, C07C 323/41

(54) **Composition cosmétique réductrice pour permanente contenant un dérivé d'acide N-(mercapto alkyl) succinamique ou de N-(mercapto alkyl) succinimide, et son utilisation dans un procédé de déformation permanente des cheveux.**

(30) Priorité : **02.07.90 FR 9008343**

(43) Date de publication de la demande : **08.01.92 Bulletin 92/02**

(45) Mention de la délivrance du brevet : **29.09.93 Bulletin 93/39**

(84) Etats contractants désignés : **AT BE CH DE DK ES FR GB GR IT LI NL SE**

(56) Documents cités :
**EP-A- 0 200 208**
**EP-A- 0 354 835**
**FR-A- 1 303 214**
**US-A- 4 888 138**

(56) Documents cités :
**PATENT ABSTRACTS OF JAPAN, vol. 12, no. 59 (C-478)[2906], 23 février 1988; & JP-A-62 205 015**
**PATENT ABSTRACTS OF JAPAN, vol. 12, no. 405 (C-539)[3252], 26 octobre 1988; & JP-A-63 146 808**

(73) Titulaire : **L'OREAL**
**14, Rue Royale**
**F-75008 Paris (FR)**

(72) Inventeur : **Maignan, Jean**
**8, rue Halévy**
**F-93290 - Tremblay Les Gonesse (FR)**
Inventeur : **Malle, Gérard**
**18, Grande rue**
**F-77580 - Villiers Sur Morin (FR)**

(74) Mandataire : **Stalla-Bourdillon, Bernard et al**
**CABINET NONY & CIE 29, rue Cambacérès**
**F-75008 Paris (FR)**

**EP 0 465 342 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet une composition cosmétique réductrice, pour le premier temps d'une opération de déformation permanente des cheveux, contenant en tant qu'agent réducteur un dérivé d'acide N-(mercapto alkyl) succinamique ou de N-(mercapto alkyl) succinimide ou leurs homologues et son utilisation dans un procédé de déformation permanente des cheveux.

La technique pour réaliser la déformation permanente des cheveux consiste, dans un premier temps, à réaliser l'ouverture des liaisons disulfures de la kératine (cystine) à l'aide d'une composition contenant un agent réducteur (étape de réduction), puis, après avoir de préférence rincé la chevelure, à reconstituer dans un second temps lesdites liaisons disulfures en appliquant, sur les cheveux sous tension, une composition oxydante, (étape d'oxydation, dite aussi de fixation) de façon a donner aux cheveux la forme recherchée. Cette technique permet indifféremment de réaliser soit l'ondulation des cheveux, soit leur défrisage ou leur décrêpage.

Les compositions pour réaliser le premier temps d'une opération de permanente se présentent généralement sous forme de lotions, de crèmes, de gels ou de poudres à diluer dans un support liquide et contiennent, en tant qu'agent réducteur, de préférence un mercaptan.

Parmi ces derniers, ceux couramment utilisés sont l'acide thioglycolique et l'acide thiolactique ou un mélange de ces acides ainsi que leurs esters par exemple le monothioglycolate de glycérol ou de glycol.

Ces agents réducteurs sont particulièrement efficaces pour réduire les liaisons disulfures de la kératine notamment l'acide thioglycolique qui peut être considéré comme le produit de référence en permanente, et qui conduit à un taux de réduction d'environ 50 %.

Ces agents réducteurs présentent cependant un inconvénient majeur dans la mesure où ils dégagent de mauvaises odeurs, d'ailleurs propres aux composés soufrés qui rendent les opérations de permanente parfois pénibles non seulement pour les personnes qui les subissent mais également pour les personnes qui les effectuent.

En vue de remédier à cet inconvénient, il est généralement fait usage d'un parfum permettant de masquer les odeurs.

Après d'importantes recherches, on a maintenant constaté, de façon tout à fait inattendue et surprenante, qu'en utilisant une nouvelle classe de dérivés d'acide N-(mercapto alkyl) succinamiques ou de N-(mercapto alkyl) succinimides ou leurs homologues, il était possible de remédier à certains inconvénients des agents réducteurs de l'état de la technique.

Les agents réducteurs des compositions selon l'invention présentent en effet de bonnes propriétés réductrices et sont pratiquement dépourvus d'odeur.

La présente invention a donc pour objet à titre de produit industriel nouveau, une composition cosmétique pour le premier temps d'une opération de déformation permanente des cheveux, contenant, dans un véhicule cosmétique approprié, en tant qu'agent réducteur, au moins un dérivé d'acide N-(mercapto alkyl) succinamique ou de N-(mercapto alkyl) succinimide ou un de leurs homologues ayant la formule générale suivante :

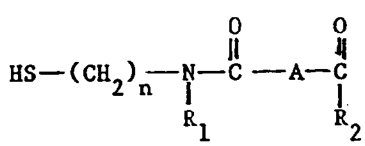 (I)

dans laquelle :

n est 2 ou 3

A représente un radical divalent choisi parmi :

(i) $-(CH_2)_m-$, m étant 2 ou 3,

(ii)

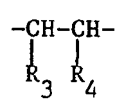 ,

$R_3$ et $R_4$, identiques ou différents, représentant un radical alkyle ayant de 1 à 4 atomes de carbone ou $R_3$ et $R_4$ forment ensemble, avec les atomes de carbone adjacents, un cycle cyclohexane, et

(iii)

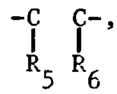

$R_5$ et $R_6$, identiques ou différents, représentant un atome d'hydrogène, un radical alkyle ayant de 1 à 4 atomes de carbone ou $R_5$ et $R_6$ forment ensemble, avec les atomes de carbone adjacents, un cycle benzénique,

$R_1$ représente un atome d'hydrogène et

$R_2$ représente sur radical hydroxy ou

$R_1$ et $R_2$ pris ensemble forment une liaison simple, et les sels d'une base organique ou minérale desdits composés sous forme acide libre.

Lorsque les composés de formule générale (I) ci-dessus se présentent sous forme de sels, il s'agit plus particulièrement d'un sel d'ammonium, d'un sel d'amine secondaire ou tertiaire ou encore d'un sel d'un métal alcalin ou alcalino-terreux.

Dans la formule générale (I) ci-dessus, lorsque les radicaux $R_3$ et $R_4$ forment ensemble avec les atomes de carbone adjacents un cycle cyclohexane, le radical divalent correspondant est le radical 1,2-cyclohexylidène.

Par ailleurs, lorsque les radicaux $R_5$ et $R_5$ forment ensemble avec les atomes de carbone adjacents un cycle benzénique, le radical divalent correspondant est le radical o-phenylène.

Par radical alkyle ayant 1 à 4 atomes de carbone, on doit entendre un radical méthyle, éthyle, propyle, isopropyle ou butyle.

Parmi les composés de formule (I) ci-dessus dans lesquels $R_1$ représente un atome d'hydrogène et $R_2$ représente un radical hydroxy, on peut notamment mentionner les suivants :

acide N-(mercapto-2 éthyl) succinamique,
acide N-(mercapto-3 propyl) succinamique,
acide N-(mercapto-2 éthyl) hexahydrophtalamique,
acide N-(mercapto-3 propyl) hexahydrophtalamique,
acide N-(mercapto-2 éthyl) phtalamique,
acide N-(mercapto-3 propyl) phtalalamique,
acide N-(mercapto-2 éthyl) glutaramique,
acide N-(mercapto-3 propyl) glutaramique,
acide N-(mercapto-2 éthyl) maléamique.

Parmi les composés de formule (I) ci-dessus dans lesquels $R_1$ et $R_2$, pris ensemble, forment une liaison simple, on peut notamment mentionner les suivants :

N-(mercapto-2 éthyl) succinimide,
N-(mercapto-3 propyl) succinimide,
N-(mercapto-2 éthyl) glutarimide,
N-(mercapto-3 propyl) glutarimide,
N-(mercapto-2 éthyl) hexahydrophtalimide,
N-(mercapto-3 propyl) hexahydrophtalimide.

Certains des composés de formule (I) sont connus et d'autres nouveaux et l'on donnera ci-après en ce qui concerne ces derniers diverses méthodes permettant de les obtenir ainsi que plusieurs exemples de préparation.

Dans les compositions selon l'invention l'agent réducteur de formule générale (I) est généralement présent à une concentration comprise entre 2 et 20 % et de préférence entre 5 et 10 % en poids par rapport au poids total de la composition réductrice.

Le pH de la composition est de préférence compris entre 4,5 et 11, et plus particulièrement entre 6 et 10 et obtenu à l'aide d'un agent alcalin tel que par exemple l'ammoniaque, la monoéthanolamine, la diéthanolamine, la triéthanolamine, un carbonate ou un bicarbonate alcalin ou d'ammonium, un hydroxyde alcalin ou à l'aide d'un agent acidifiant tel que par exemple l'acide chlorhydrique, l'acide acétique, l'acide lactique, l'acide oxalique ou l'acide borique.

La composition réductrice peut également contenir d'autres agents réducteurs connus tels que par exemple l'acide thioglycolique, le monothioglycolate de glycérol ou de glycol, la cystéamine et ses dérivés acylés $C_1$-$C_4$ tels que la N-acétyl cystéamine ou la N-propionylcystéamine, la cystéine, la N-acétylcystéine, les N-mercaptoalkylamides de sucres tels que le N-(mercapto-2-éthyl) gluconamide, l'acide β-mercaptopropionique et ses dérivés, l'acide thiolactique, l'acide thiomalique, la pantéthéine, le thioglycérol, les sulfites et bisulfites d'un métal alcalin ou alcalino-terreux, les N-(mercaptoalkyl) ω-hydroxyalkyl amides décrits dans la demande de brevet EP 354 835, les N-mono ou N,N dialkylmercapto 4-butyramides décrits dans la demande de brevet EP 368 763, et les aminomercaptoalkylamides décrits dans la demande de brevet EP 403 267.

La composition réductrice peut également contenir divers ingrédients tels que par exemple des polymères cationiques tels que ceux utilisés dans les compositions des brevets français n° 79.32078, 80.26421 et 89.16273 ou encore des polymères cationiques du type ionène tels que ceux utilisés dans les compositions du brevet français n° 82.17364, des agents adoucissants et notamment des ammoniums quaternaires dérivés de la lanoline, des hydrolysats de protéines, des cires, des agents opacifiants, des parfums, des colorants, des tensio-actifs non-ioniques ou cationiques, des agents traitants ou encore des agents de pénétration tels que l'urée, la pyrrolidone ou la thiamorpholinone.

La composition réductrice selon l'invention peut être également du type exothermique, c'est à dire provoquant un certain échauffement lors de l'application sur les cheveux, ce qui apporte un agrément à la personne qui subit le premier temps de la permanente ou du défrisage.

Le véhicule des compositions selon l'invention est de préférence de l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcool gras, etc...

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent être également selon un mode particulier de réalisation sous forme dite "auto-neutralisante" ou encore "auto-régulée" et dans ce cas le composé réducteur de formule (I) est associé à au moins un disulfure soit connu pour son utilisation dans une composition réductrice pour permanente auto-neutralisante, soit dérivant d'un composé de formule (I) celui-ci pouvant être représenté par la formule générale suivante :

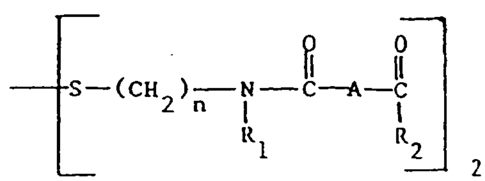

(II)

dans laquelle :

n, $R_1$, $R_2$ et A ont les mêmes significations que celles données pour la formule (I).

Parmi les disulfures connus, on peut notamment mentionner l'acide dithioglycolique, le dithioglycérol, la cystamine, la N,N-diacétyl-cystamine, la cystine, la pantéthine et les disulfures des N-(mercaptoalkyl) ω-hydroxyalkylamides décrits dans la demande de brevet EP 354 835, les disulfures des N-mono ou N,N-dialkylmercapto 4-butyramides décrits dans la demande de brevet EP 368 763 et les disulfures des aminomercaptoalkylamides décrits dans la demande de brevet EP 403 267.

Parmi les disulfures de formule (II) on peut notamment mentionner les suivants :

bis [N-(carboxy-3 propionyl) N-éthyl] disulfure,

bis [N-(carboxy-2 cyclohexane carbonyl) N-éthyl] disulfure,

bis [N-éthyl succinimide] disulfure.

Le disulfure est généralement présent dans les compositions en une proportion molaire par rapport au thiol de formule (I) allant de 0,5 à 2,5 et de préférence de 1 à 2 (voir brevet US 3.768.490).

La présente invention a également pour objet un procédé de déformation permanente des cheveux consistant, dans une première étape, à réduire les liaisons disulfures de la kératine par application, pendant environ 5 à 60 minutes, d'une composition réductrice telle que définie ci-dessus puis dans une seconde étape à reformer lesdites liaisons par application d'une composition oxydante ou éventuellement en laissant agir l'oxygène de l'air.

La présente invention a entre outre pour objet un procédé d'ondulation des cheveux dans lequel on applique une composition réductrice telle que définie ci-dessus sur des cheveux mouillés préalablememt roulés sur des rouleaux ayant de 4 à 20 mm de diamètre, la composition pouvant éventuellement être appliquée au fur et à mesure de l'enroulage des cheveux ; on laisse ensuite agir la composition réductrice pendant un temps de 5 à 60 min, de préférence de 5 à 30 min, puis on rince abondamment après quoi on applique, sur les cheveux enroulés, une composition oxydante permettant de reformer les liaisons disulfures de la kératine pendant un temps de pose de 2 à 10 min. Après avoir enlevé les rouleaux, on rince abondamment la chevelure.

La composition d'oxydation ou oxydante est du type couramment utilisé et contient comme agent oxydant

de l'eau oxygénée, un bromate alcalin, un persel, un polythionate ou un mélange de bromate alcalin et de persel. Cette oxydation peut être immédiate ou différée. La concentration en eau oxygénée peut varier de 1 à 20 volumes et de préférence de 1 à 10, la concentration en bromate alcalin de 2 à 12 % et celle en persel de 0,1 à 15 % en poids par rapport au poids total de la composition oxydante. Le pH de la composition oxydante est généralement compris entre 2 et 10.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec un peigne à larges dents, avec le dos d'un peigne ou à la main. Après un temps de pose de 5 à 60 min, en particulier de 5 à 30 min, on procède alors à un nouveau lissage puis on rince soigneusement et on applique la composition oxydante ou fixatrice que l'on laisse agir pendant 2 à 10 min environ puis on rince abondamment les cheveux.

Les composés de formule générale (I) sont préparés selon des procédés connus en soi.

Les dérivés d'acide N-(mercapto alkyl) succinamique ou leurs homologues, c'est à dire les composés de formule (I) dans laquelle $R_1$ = H et $R_2$ = OH, sont obtenus en faisant réagir une mercapto-alkylamine (1) sur un anhydride de formule (2) selon le schéma réactionnel suivant :

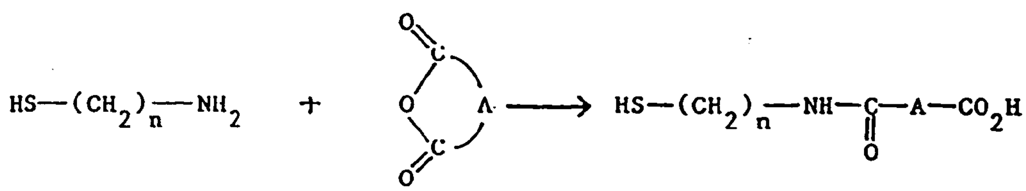

(1) (2) (Ia)

Les mercapto alkylamines (1) utilisées sont soit la mercapto-2 éthylamine ou cystéamine (n=2) soit la mercapto-3 propylamine (n=3).

Les anhydrides (2) sont l'anhydride succinique, l'anhydride maléique, l'anhydride 1,2 cyclohexane dicarboxylique, l'anhydride phtalique, l'anhydride méthyl succinique, l'anhydride glutarique.

La réaction d'ouverture de l'anhydride (2) est généralement conduite sous atmosphère inerte dans un alcool tel que le méthanol, l'éthanol, l'isopropanol ou le butanol, et suivant le point d'ébullition de ce dernier et la facilité avec laquelle les acides de formule (Ia) se cyclisent, à un température comprise entre 0 et 110 °C.

Il peut être avantageux d'utiliser des sels des mercapto alkylamines (1) notamment les chlorhydrates, et dans ce cas le solvant préféré est l'éthanol et la base utilisée pour libérer l'amine est une amine tertiaire. Il peut être avantageux d'ajouter un excès d'amine, ceci afin de salifier au fur et à mesure l'acide (Ia) formé et ainsi limiter la cyclisation.

Lorsque les mercapto alkylamines (1) sont sous forme amine libre, il peut être avantageux de ne pas utiliser de solvant et mélanger dans des proportions stoechiométriques la mercapto alkylamine et l'anhydride, et porter le mélange à une température comprise entre 20 et 100 °C.

Lorsque la mercapto alkylamine est la cystéamine, il est souhaitable d'opérer dans un autoclave ceci afin d'éliminer les problèmes de sublimation.

L'évolution de la réaction est suivie par dosage de la mercapto alkylamine non transformée. Lorsque le temps réactionnel est trop long, il peut être avantageux d'utiliser un excès de la mercapto alkylamine que l'on élimine en fin de réaction par filtration du mélange sur une résine sulfonique acide.

Les dérivés de N-(mercapto alkyl) succinimide ou leurs homologues, c'est à dire les composés de formule (I) dans laquelle $R_1$ et $R_2$, pris ensemble, forment une liaison simple, sont obtenus pas déshydratation des acides succinamiques de formule (Ia).

Afin de réaliser la réaction de déshydratation, on choisit de préférence un solvant aromatique formant un azéotrope avec l'eau formée au cours de la réaction, ayant un point d'ébullition suffisamment élevé tel que le toluène ou le xylène. On utilise dans ce cas un séparateur d'eau (Dean Stark) et il est alors facile de suivre l'évolution de la réaction en mesurant le volume d'eau éliminé.

Les N-(mercapto alkyl) succinimides ou leurs homologues, peuvent être également obtenus à partir de la mercapto alkylamine (1) et de l'anhydride (2) en présence ou non de solvant, sans isoler intermédiairement le dérivé d'acide succinamique (Ia), la cyclisation étant effectuée par chauffage prolongé en plaçant le mélange sous pression réduite lorsque l'on n'utilise pas de solvant réactionnel.

Si au cours des réactions un certaine quantité de thiol est oxydée en disulfure correspondant, le mélange

réactionnel est alors dilué par deux fois son volume d'eau et est agité en présence d'un mélange de résine sulfonique et de poudre de zinc pendant 3 à 10 heures.

La majorité du disulfure étant réduite, le mélange est alors filtré et on obtient une solution du composé attendu qui peut être utilisé directement.

Dans le cas de compositions auto-neutralisantes, le mélange réactionnel contenant une certaine quantité de thiol oxydé en disulfure peut-être utilisé sans procéder à la réduction du disulfure.

Dans certains cas, suivant la nature de l'anhydride (2) utilisé, les acides succinamiques (Ia) se cyclisent facilement et on peut obtenir un mélange de l'acide et de l'imide correspondant.

Les disulfures (II) selon l'invention peuvent être obtenus par oxydation des composés de formule (I) soit à l'air libre soit de préférence en utilisant par exemple l'eau oxygénée en présence éventuellement d'ions ferreux.

Les disulfures (II) peuvent également être obtenus par réaction d'un disulfure de formule (3) sur un anhydride (2) selon le schéma réactionnel suivant :

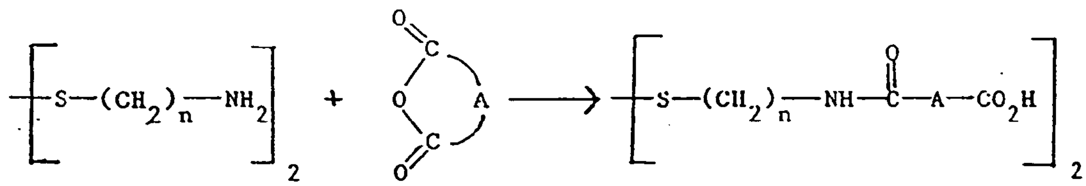

(3) (2) (IIa)

(IIa)

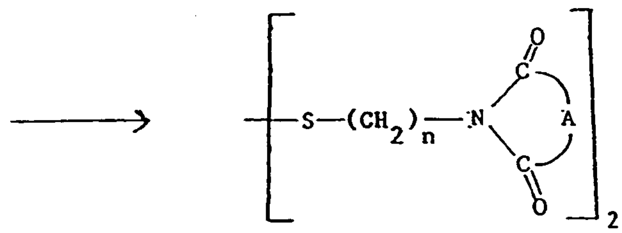

(IIb)

Les disulfures (IIa) conduisent par chauffage prolongé aux disulfures de formule (IIb).

La présente invention a également pour objet à titre de produits industriels nouveaux les composés répondant aux formules générales (III) et (IV) suivantes :

A.

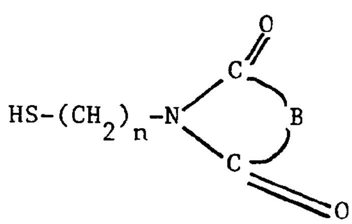

(III)

dans laquelle :

n est 2 ou 3, et

B représente un radical divalent pris dans le groupe constitué par :

(i) $-(CH_2)_3-$

(ii) 1,2-cyclohexylidène, et

(iii) -CH=CH- lorsque dans ce dernier cas n est 3 et leurs disulfures correspondants.

B. $HS-(CH_2)_n-NH-\overset{O}{\overset{\|}{C}}-Z-CO_2H$ (IV)

dans laquelle :

n est 2 ou 3

(a) lorsque n est 2

Z représente le radical 1,2-cyclohexylidène

(b) lorsque n est 3

Z est un radical divalent choisi dans le groupe constitué par :

(i) $-(CH_2)_m-$, m étant 2 ou 3,

(ii) -CH=CH-

(iii) 1,2-cyclohexylidène, et

(iv) o-phénylène,

et leurs disulfures correspondants, ainsi que le disulfure correspondant lorsque n est 2 et Z représente un radical o-phénylène.

On va maintenant donner à titre d'illustration plusieurs exemples de préparation des composés de formules (I) et (II) ainsi que plusieurs exemples des compositions réductrices selon l'invention et leur utilisation dans un procédé de déformation permanente des cheveux.

## EXEMPLE I

### Préparation de l'acide N-(mercapto-2 éthyl) succinamique

a) A partir du chlorhydrate de cystéamine

Dans un réacteur on introduit sous atmosphère inerte 114g de chlorhydrate de cystéamine auquel on ajoute, goutte à goutte, sous agitation mécanique et à température ambiante, 133 $cm^3$ de triéthanolamine dissous dans 180 $cm^3$ d'éthanol absolu. On observe une légère exothermicité et on introduit ensuite 100 g d'anhydride succinique de façon à maintenir le milieu à une température voisine de 40°C. Le mélange est alors agité 3 heures à température ambiante et abandonné une nuit sous atmosphère inerte. La transformation de l'anhydride est suivie par chromatographie phase vapeur (C.P.V.) et celle de l'amine de départ par dosage de la quantité d'amine restante. Après 3 heures de mise en réaction, il ne reste pratiquement plus de produit de départ. Le sel est éliminé par filtration et le filtrat est concentré sous pression réduite. On obtient 170 g d'un produit brut qui cristallise sous forme d'un solide pâteux à température ordinaire. Ce produit est agité sous atmosphère inerte dans 200 $cm^3$ de dichloro-1,2 éthane que l'on porte progressivement à ébullition. Après retour à température ambiante, on observe deux phases liquides et on refroidit à 0°C pendant 4 heures. Le solide formé est essoré, lavé trois fois avec 100 $cm^3$ d'éther éthylique puis séché sous pression réduite. On obtient 125 g d'une poudre blanche que l'on recristallise dans l'acétate d'éthyle à chaud. Après refroidissement, essorage et séchage sous vide à 45°C, on obtient 110 g d'acide N-(mercapto-éthyl) succinamique sous forme d'un solide blanc de point de fusion : 82°C. Le spectre R.M.N.$^1$H 250 MHz est conforme à la structure attendue. Les dosages des fonctions thiol et acide carboxylique sort supérieurs à 95 % de la théorie.

Analyse élémentaire : $C_6H_{11}NO_3S$

| | | | | | |
|---|---|---|---|---|---|
| Calc. | C : 40,66 | H : 6,26 | N : 7,90 | O : 27,08 | S : 18,09 |
| Tr. | 40,79 | 6,24 | 7,91 | 27,24 | 17,89 |

b) A partir de la cystéamine

Un mélange de 5 g de cystéamine et de 6 g d'anhydride succinique est agité sous atmosphère inerte à une température d'environ 100°C. La transformation des produits de départ est pratiquement totale une demi-heure après le début de la réaction. Le mélange est solubilisé dans 50 $cm^3$ d'eau auquel on ajoute sous agitation 5 g de résine sulfonique acide et 3 g de poudre de zinc pour transformer le disulfure présent dans le mélange . La résine est éliminée par filtration et le filtrat est concentré sous pression réduite. On obtient après séchage 9 g d'acide N-(mercapto-2 éthyl) succinamique dont les caractéristiques sont identiques à celles de l'acide obtenu suivant le mode opératoire (a) ci-dessus.

EXEMPLE II

Préparation de la N-(mercapto-2 éthyl) succinimide

a) A partir de la cystéamine

On porte progressivement sous atmosphère inerte un mélange agité de 5 g de cystéamine et de 6 g d'anhydride succinique pendant 6 heures à une température d'environ 130°C. Une demi-heure environ est nécessaire pour la transformation totale de l'anhydride succinique (C.P.V.). A ce stade, pour faciliter la réaction de cyclisation, on place le milieu réactionnel sous pression réduite et l'évolution du mélange réactionnel est suivie par dosage acidimétrique de l'acide N-(mercapto-2 éthyl) succinamique non transformé. A la fin de la réaction, le mélange est refroidit et on obtient 7 g de produit brut que l'on dissout dans 50 cm³ d'eau auquel on ajoute sous agitation 10 g de résine sulfonique acide. Après une heure d'agitation, la résine ayant fixé les traces de cystéamine ou de cystamine n'ayant pas réagi, est éliminée par filtration. Pour réduire le disulfure éventuellement présent, on ajoute au filtrat agité, 10 g de résine sulfonique et 3 g de poudre de zinc. Après 3 heures le mélange est filtré et le filtrat est concentré sous pression réduite. Après séchage, on obtient 5 g de N-(mercapto-2 éthyl) succinimide sous forme d'une poudre blanche, de point de fusion : 45°C. Les spectres $^1H$ et $^{13}C$ R.M.N. sont conformes à la structure attendue.

Analyse élémentaire : $C_6H_{11}NO_3S$

| | | | | | |
|---|---|---|---|---|---|
| Calc. | C : 45,27 | H : 5,70 | N: 8,80 | O : 20,10 | S : 20,14 |
| Tr. | 45,32 | 5,75 | 8,64 | 20,10 | 20,14 |

b) A partir de l'acide N-(mercapto-2 éthyl) succinamique

Dans un réacteur de 100 cm³ muni d'un séparateur d'eau (Dean Stark) on porte un mélange, agité sous atmosphère inerte de 20 g d'acide N-(mercapto-2 éthyl) succinamique dans 50 cm³ de xylène, à une température de 140°C. Après environ 5 heures de réaction, la quantité d'eau théorique (2 cm³) est éliminée. Le xylène est alors distillé sous pression réduite. Le produit brut est séché et on obtient 18 g d'un produit pâteux qui cristallise lentement à température ambiante, ses caractéristiques sont les mêmes que celles du produit préparé suivant le mode opératoire (a) ci-dessus.

EXEMPLE III

Préparation du bis [N-(carboxy-3 propionyl)N-éthyl ] disulfure

Une solution de 3,7 g de cystéamine et de 2 g d'anhydride succinique dans 80 cm³ d'éthanol est portée sous agitation à la température d'ébullition de l'éthanol pendant 10 heures, temps au bout duquel tout l'anhydride est transformé. Le mélange est abandonné une nuit et le lendemain, on fait barboter, à température ordinaire, de l'air au sein de la solution jusqu'à ce que la totalité du thiol soit transformé en disulfure. Le mélange est concentré à mi-volume puis l'on refroidit à O°C. Le disulfure cristallisé est essoré et séché. On obtient 2 g de bis N-(carboxy-3 propionyl)N-éthyl disulfure sous forme d'une poudre blanche de point de fusion : 141°C.

Analyse élémentaire : $C_{12}H_{20}N_2O_6S_2$

| | | | | | |
|---|---|---|---|---|---|
| Calc. | C : 40,89 | H : 5,72 | N : 7,95 | O : 27,24 | S : 18,20 |
| Tr. | 40,64 | 5,77 | 7,95 | 27,3 | 18,00 |

EXEMPLE IV

Préparation du bis[(N-éthyl succinimide)] disulfure

On dissout 15,9 g (0,1 mole) de N-(mercapto-2 étyl) succinimide, décrit à l'ensemble II, dans environ 200 cm³ d'éthanol et 200 cm³ d'eau. On refroidit à une température comprise entre 15 et 20°C, ajoute quelques gouttes d'ammoniaque à 20 % pour rendre le pH alcalin, puis goutte à goutte de l'eau oxygénée à 110 volumes jusqu'à transformation totale du thiol en disulfure (dosage à l'iode).

La solution est clarifiée par filtration sur verre fritté puis concentrée sous pression réduite à environ 100

$cm^3$. Après refroidissement à O°C, le solide blanc est essoré puis dissous à nouveau dans le minimum d'éthanol. La solution est filtrée puis concentrée sous vide jusqu'à début de cristallisation. On refroidit à + 5°C, essore les cristaux et sèche sous vide à 60-70°C. On obtient 12,8 g de bis (N-éthyl succinimide) disulfure sous la forme d'un solide blanc de point de fusion : 119°C.

Le spectre RMN$^1$H 80MHz est conforme à la structure attendue.

Analyse élémentaire : $C_{12}H_{16}N_2O_4S_2$

| | C | H | N | O | S |
|---|---|---|---|---|---|
| Calc. | 44,55 | 5,10 | 8,85 | 20,23 | 20,27 |
| Tr. | 45,66 | 5,03 | 8,78 | 20,40 | 20,21 |

## EXEMPLE V

### Préparation de l'acide N-(mercapto-2éthyl)hexahydrophtalamique

A une solution de 7,72 g (0,1 mole) de cystéamine dans 40 $cm^3$ de méthanol, maintenue sous atmosphère inerte et refroidie à une température comprise entre 0 et 5°C, on ajoute par portions 15,43 g (0,1 mole) d'anhydride hexahydrophtalique. Après 7 heures d'agitation à cette température, il ne reste plus que des traces de cystéamine de départ. La solution est évaporée à sec sous pression réduite à température ambiante. On obtient 23 g d'acide N-(mercapto-2 éthyl) hexahydrophtalamique sous la forme d'une huile incolore.

Le spectre RMN$^1$H 80 MHz correspond à la structure attendue.

## EXEMPLE VI

### Préparation du N-(mercapto-2 éthyl) hexahydrophtalimide

Dans un réacteur muni d'un Dean-StarK , on introduit sous gaz inerte 15,43 g (0,2 mole) de cystéamine, 100 $cm^3$ de toluène et 30,83 g (0,2 mole) d'anhydride hexahydrophtalique puis on chauffe au reflux sous agitation avec distillation de l'eau formée. Après 4 heures de reflux, la réaction est complète. On évapore à sec sous pression réduite. Le solide blanc obtenu est purifié par recristallisation dans l'acétate d'éthyle. On obtient, après séchage sous vide à 70°C, 37,5 g de N-(mercapto-2 éthyl) hexahydrophtalimide sous la forme d'un solide blanc de point de fusion : 79-80°C.

Le spectre RMN$^1$H 80 MHz est conforme à la structure attendue.

Analyse élémentaire : $C_{10}H_{15}NO_2S$

| | C | H | N | O | S |
|---|---|---|---|---|---|
| Calc. | 56,31 | 7,09 | 6,57 | 15,00 | 15,03 |
| Tr. | 56,07 | 7,11 | 6,39 | 15,02 | 14,93 |

## EXEMPLE VII

### Préparation de l'acide N-(mercapto-3 propyl) glutaramique

A une solution de 6,84 g (0,075 mole) de mercapto-3 propylamine (homocystéamine) dans 20 $cm^3$ de méthanol, maintenue sous atmosphère inerte et agitée à température ambiante, on ajoute par portions 8,56 g (0,075 mole) d'anhydride glutarique en maintenant la température du milieu inférieur à 40°C. Après 6h30 d'agitation la réaction est complète. La solution est évaporée à sec sous pression réduite. On sèche ensuite sous vide à température inférieure à 60°C et obtient 19,5 g d'acide N-(mercapto-3 propyl) glutaramique sous la forme d'une huile incolore.

Le spectre RMN$^1$H 80 MHz est conforme à la structure attendue.

EXEMPLES DE COMPOSITION

EXEMPLE 1 :

On prépare selon l'invention une composition réductrice de déformaticn permanente des cheveux en procédant au mélange des ingrédients suivants :

Acide N-(mercapto-2 éthyl) succinamique.............. 18 g
Monoethanolamine qsp.........pH 8,5
Chlorure d'oléocetyl diméthyl hydroxyéthyl ammonium
vendu sous la dénomination de "CHIMEXANE CI" par
la Société CHIMEX.................................... 0,3 g
Parfum
Conservateur
Eau déminéralisée qsp................................ 100 g

Cette composition est appliquée sur des cheveux mouillés préalablement enroulés sur des rouleaux de mise en plis. Après avoir laissé agir la composition pendant environ 15 min., on rince abondamment à l'eau puis on applique la composition oxydante suivante :

Eau oxygénée qsp...................... 8 volumes
Stabilisants
Oxyde de lauryl diméthyl amine ..................... 0,7 g
Parfum
Acide lactique qsp............pH 3.0
Eau déminéralisée qsp.............................. 100 g

On laisse agir la composition oxydante pendant environ 10 min., puis on enlève les rouleaux et rince abondamment la chevelure à l'eau.

Après séchage sous casque, les cheveux présentent de belles boucles.

Dans cet exemple, la composition oxydante peut être remplacée par la suivante :

Eau oxygénée qsp..................... 8 volumes
Stabilisants
Parfum
Acide citrique qsp............pH 3,5
Eau déminéralisée qsp............................. 100 g

EXEMPLE 2

Selon le même mode de réalisation qu'à l'exemple 1, en a procédé à une déformation permanente des cheveux à l'aide des compositions réductrices et oxydantes suivantes :

A. Composition réductrice

Acide N-(mercapto-2 éthyl) succinamique.............. 13 g
bis N-(carboxy-3 propionyl)N éthyl disulfure........ 5 g
Monoéthanolamine qsp........... pH 9.0
Cocoylamidopropyl betaine avec
monoglycéride de coprah vendu sous la dénomination de
"TEGOBETAINE HS" par la Société GOLDSCHMIDT.......... 0,3 g
Parfum
Conservateur
Eau déminéralisée qsp............................ 100 g

B. Composition oxydante

Bromate de sodium .................................. 8 g
Triéthanolamine qsp...................... pH 8.0
Phosphate monosodique hydraté (12 $H_2O$)............ 0,3 g
Phosphate trisodique hydraté (2 $H_2O$)............. 0,5 g
Cocoylamidopropyl betaine avec monoglycéride de coprah
vendu sous la dénomination de "TEGOBETAINE HS" par
la Société GOLDSCHMIDT.............................. 1 g
Eau déminéralisée qsp............................ 100 g

EXEMPLE 3 :

A. Composition réductrice

N-(mercapto-2 éthyl) succinimide..................... 10 g
Monoéthanolamine qsp...................... pH 9.0
Chlorure d'oléocetyl diméthyl hydroxyéthyl ammonium
vendu sous la dénomination de "CHIMEXANE CI" par la
Société CHIMEX...................................... 0,3 g
Parfum
Conservateur
Eau déminéralisée qsp............................ 100 g

B. Composition oxydante

| | | |
|---|---|---|
| Eau oxygénée à 200 volumes | | 4,8 g |
| Sulfate d'hydroxy-8 quinoleine | | 0,01 g |
| Phénacétine | | 0,05 g |
| Acide citrique | qsp | pH 3.0 |
| Parfum | | |
| Eau déminéralisée | qsp | 100 g |

EXEMPLE 4

A. Composition réductrice

| | | |
|---|---|---|
| Acide N-(mercapto-2 éthyl) succinamique | | 6 g |
| N-(mercapto-2 éthyl) succinimide | | 4 g |
| bis N-(carboxy-3 propionyl)N éthyl disulfure | | 2 g |
| Monoéthanolamine | qsp | pH 8.7 |
| Chlorure de cetyl triméthyl ammonium | | 1,0 g |
| Parfum | | |
| Conservateur | | |
| Eau déminéralisée | qsp | 100 g |

B. Composition oxydante

| | | |
|---|---|---|
| Eau oxygénée | qsp | 8 volumes |
| Mélange de lauryl et de myristyl éther sulfate de sodium (dans un rapport 70/30 étherifié à l'aide de 2,2 moles d'oxyde d'éthylène) à 25% vendu sous la dénomination de "SIPON AOS 225" par la Société HENKEL | | 3,5 g |
| Stabilisants | | |
| Parfum | | |
| Acide citrique | qsp | pH 3,0 |
| Eau déminéralisée | qsp | 100 g |

EXEMPLE 5

A. Composition réductrice

Acide N-(mercapto-2 éthyl) hexahydrophtalamique...... 11,5 g
Monoéthanolamine qsp...................... pH 9.0
Chlorure d'oléocetyl diméthyl hydroxyéthyl ammonium vendu sous la dénomination de "CHIMEXANE CI" par la Société CHIMEX.................................... 0,3 g
Parfum
Conservateur
Eau déminéralisée qsp............................ 100 g

B. Composition oxydante

Eau oxygénée à 200 volumes.......................... 4,8 g
Sulfate d'hydroxy-8 quinoleine...................... 0,01 g
Phénacétine......................................... 0,05 g
Acide citrique qsp...................... pH 3.0
Parfum
Eau déminéralisée qsp............................ 100 g

EXEMPLE 6 :

A. Composition réductrice

N-(mercapto-2 éthyl) hexahydrophtalimide............ 16 g
Monoéthanolamine qsp...................... pH 9.0
Chlorure d'oléocetyl diméthyl hydroxyéthyl ammonium vendu sous la dénomination de "CHIMEXANE CI" par la Société CHIMEX.................................... 0,3 g
Parfum
Conservateur
Eau déminéralisée qsp............................ 100 g

B. Composition oxydante

Eau oxygénée à 200 volumes.......................... 4,8 g
Sulfate d'hydroxy-8 quinoleine...................... 0,01 g
Phénacétine......................................... 0,05 g
Acide citrique qsp...................... pH 3.0
Parfum
Eau déminéralisée qsp............................100 g

EXEMPLE 7

A. Composition réductrice

- Acide N-(mercapto-2 éthyl)succinamique 9,3 g
- Copolymère de N-vinylpyrrolidone/méthacrylate de diméthylaminoéthyle en solution aqueuse à 20 % en poids vendu par la société GAF sous la dénomination "Copolymer 845" .................................. 1 g
- Ammoniaque qsp .................... pH 9.0
- Parfums qs
- Colorant qs
- Conservateur qs
- Eau qsp ............................ 100g

B. Composition oxydante

- Eau oxygénée à 200 volumes ....................... 4,8g
- Acide citrique qsp .................... pH 3,0
- Parfum
- Eau déminéralisée qsp ............................ 100g

EXEMPLE 8

A. Composition réductrice

- Cystéine ........................................ 2,50g
- Acide N-(mercapto-2 éthyl) succinamique ........... 3,63g
- Chlorure d'oléocétyl diméthyl hydroxyéthyl ammonium vendu sous la dénomination de "CHIMEXANE CI" par la Société CHIMEX ............................. 1,30g
- Monoéthanolamine qsp ................... pH 9
- Eau déminéralisée qsp ............................ 100g

B. Composition oxydante

- Eau oxygénée à 200 volumes ....................... 4,8g
- Acide citrique qsp ................... pH 3,0
- Parfum
- Eau déminéralisée qsp ............................ 100g

EXEMPLE 9

A. Composition réductrice

- Cystéine ........................................ 1,34g
- Acide N-(mercapto-2 éthyl) succinamique ............ 5,31g
- Chlorure d'oléocétyl diméthyl hydroxyéthyl ammonium vendu sous la dénomination de "CHIMEXANE CI" par la Société CHIMEX ................................ 130g
- Monoéthanolamine qsp .................... pH 9
- Eau déminéralisée qsp ............................ 100g

B. Composition oxydante

- Eau oxygénée à 200 volumes ....................... 4,8g
- Acide citrique qsp ................... pH 3,0
- Parfum
- Eau déminéralisée qsp ............................ 100g

EXEMPLE 10

A. Composition réductrice

- Cystéine ........................................ 5,0g
- Acide N-(mercapto-2 éthyl) succinamique ............ 3,63g
- Chlorure d'oléocétyl diméthyl hydroxyéthyle ammonium vendu sous la dénomination de "CHIMEXANE CI" par la Société CHIMEX .................................. 1,30g
- Monoéthanolamine qsp .................... pH 9
- Eau déminéralisée qsp ............................ 100g

B. Composition oxydante

- Eau oxygénée à 200 volumes ....................... 4,8g
- Acide citrique qsp ................... pH 3,0
- Parfum
- Eau déminéralisée qsp ............................ 100g

EXEMPLE 11

A. Composition réductrice

- Chlorhydrate de cystéamine ........................ 2,77g
- Acide N-(mercapto-2 éthyl) succinamique ........... 7,26g
- Chlorure d'oléocétyl diméthyl hydroxyéthyl ammonium vendu sous la dénomination de "CHIMEXANE CI" par la Société CHIMEX .................................... 1,30g
- Monoéthanolamine qsp .................... pH 9
- Eau déminéralisée qsp ............................ 100g

B. Composition oxydante

- Eau oxygénée à 200 volumes ....................... 4,8g
- Acide citrique qsp .................. pH 3,0
- Parfum
- Eau déminéralisée qsp ............................ 100g

## Revendications

1. Composition cosmétique pour le premier temps d'une opération de déformation permanente des cheveux caractérisée par le fait qu'elle contient, dans un véhicule cosmétique approprié, en tant qu'agent réducteur, au moins un dérivé d'acide N-(mercapto alkyl) succinamique ou de N-(mercapto alkyl) succinimide ou un de leurs homologues ayant la formule générale suivante :

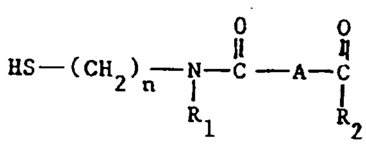
(I)

dans laquelle :

n est 2 ou 3

A représente un radical divalent choisi parmi :

(i) $-(CH_2)_m-$, m étant 2 ou 3,

(ii)

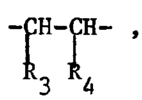

$R_3$ et $R_4$, identiques ou différents, représentant un radical alkyle ayant de 1 à 4 atomes de carbone ou $R_3$ et $R_4$ forment ensemble, avec les atomes de carbone adjacents, un cycle cyclohexane, et

(iii)

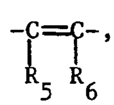

$R_5$ et $R_6$, identiques ou différents, représentant un atome d'hydrogène, un radical alkyle ayant de 1 à 4 atomes de carbone ou $R_5$ et $R_6$ forment ensemble, avec les atomes de carbone adjacents, un cycle benzénique,

$R_1$ représente un atome d'hydrogène et
$R_2$ représente un radical hydroxy ou
$R_1$ et $R_2$ pris ensemble forment une liaison simple,
et les sels d'une base organique ou minérale desdits composés sous forme acide libre.

2. Composition selon la revendication 1, caractérisée par le fait que les sels, des composés de formule (I) sous forme acide libre, sont ceux d'ammonium, d'une amine secondaire ou tertiaire, ou d'un métal alcalin ou alcalino-terreux.

3. Composition selon la revendication 1 ou 2, caractérisée par le fait que lorsque dans la formule générale (I) $R_1$ représente un atome d'hydrogène et $R_2$ représente un radical hydroxy, les composés sont choisis parmi :
acide N-(mercapto-2 éthyl) succinamique,
acide N-(mercapto-3 propyl) succinamique,
acide N-(mercapto-2 éthyl) hexahydrophtalamique,
acide N-(mercapto-3 propyl) hexahydrophtalamique,
acide N-(mercapto-2 éthyl) phtalamique,
acide N-(mercapto-3 propyl) phtalamique,
acide N-(mercapto-2 éthyl) glutaramique,
acide N-(mercapto-3 propyl) glutaramique et,
acide N-(mercapto-2 éthyl) maléamique.

4. Composition selon l'une quelconque des revendications 1 et 2, caractérisée par le fait que lorsque dans la formule générale (I) $R_1$ et $R_2$ pris ensemble forment une liaison simple, les composés sont choisis parmi :
N-(mercapto-2 éthyl) succinimide,
N-(mercapto-3 propyl) succinimide,
N-(mercapto-2 éthyl) glutarimide,
N-(mercapto-3 propyl) glutarimide,
N-(mercapto-2 éthyl) hexahydrophtalimide et,
N-(mercapto-3 propyl) hexahydrophtalimide.

5. Composition selon l'une quelconque des revendications précédentes, caractérisée par le fait que le composé de formule (I) est présent à une concentration comprise entre 2 et 20 % et de préférence entre 5 et 10 % en poids par rapport au poids total de la composition.

6. Composition selon l'une quelconque des revendications précédentes, caractérisée par le fait qu'elle présente un pH compris entre 4,5 et 11 et de préférence entre 6 et 10, obtenu à l'aide d'un agent alcalin choisi parmi l'ammoniaque, la monoéthanolamine, la diéthanolamine, la triéthanolamine, un carbonate ou un bicarbonate alcalin ou d'ammonium, un hydroxyde alcalin ou à l'aide d'un agent acidifiant choisi parmi l'acide chlorhydrique, l'acide acétique, l'acide lactique, l'acide oxalique ou l'acide borique.

7. Composition selon l'une quelconque des revendications précédentes, caractérisée par le fait qu'elle contient en outre au moins un autre agent réducteur choisi parmi l'acide thioglycolique, le monothioglycolate de glycérol ou de glycol, la cystéamine et ses dérivés acylés $C_1$-$C_4$, la cystéine, la N-acétylcystéine, les N-mercaptoalkylamides de sucres, l'acide, β-mercaptopropionique et ses dérivés, l'acide thiolactique, l'acide thiomalique, la pantéthéine, le thioglycérol, les sulfites et bisulfites d'un métal alcalin ou alcalino-terreux, les N-(mercaptoalkyl) ω-hydroxylamides, les N-mono ou N,N-dialkylmercapto 4-butyramides et les aminomercaptoalkylamides.

8. Composition selon l'une quelconque des revendications précédentes, caractérisée par le fait qu'elle contient, en outre, au moins un polymère cationique, un agent adoucissant, un hydrolysat de protéines, une cire, un agent opacifiant, un parfum, un colorant, un agent tensio-actif non-ionique ou cationique, un agent traitant ou encore un agent de pénétration.

9. Composition selon l'une quelconque des revendications précédentes, caractérisée par le fait qu'elle contient en outre au moins un disulfure, la composition étant auto-neutralisante.

**10.** Compositlon selon la revendication 9, caractérisée par le fait que le disulfure est choisi parmi l'acide dithioglycolique, le dithioglycérol, la cystamine, le N,N-diacétyl-cystamine, la cystine, la pantéthine, les disulfures des N-(mercaptoalkyl) ω-hydroxyalkylamides, les disulfures des N-mono ou N,N-dialkylmercapto 4-butyramides et les disulfures des aminomercapto-alkylamides

**11.** Composition selon la revendication 9, caractérisée par le fait que le disulfure est un disulfure, d'un composé de formule générale (I), correspondant à la formule générale suivante :

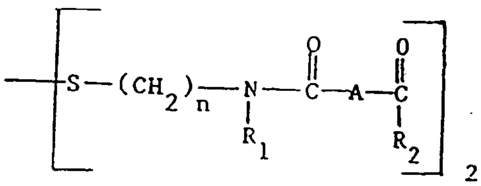

(II)

dans laquelle n, $R_1$, $R_2$, et A ont les mêmes significations que celles données à la revendication 1.

**12.** Composition selon la revendication 11, caractérisée par le fait que le disulfure de formule (II) est choisi parmi :

bis [N-(carboxy-3 propionyl) N-éthyl] disulfure,
bis [N-(carboxy-2 cyclohexane carbonyl) N-éthyl] disulfure,
bis [N-éthyl succinimide ] disulfure.

**13.** Composition selon l'une quelconque des revendications 9 à 12, caractérisée par le fait que le disulfure est présent en une proportion molaire par rapport au composé de formule (I) allant de 0,5 à 2,5 et de préférence de 1 à 2.

**14.** Procédé de déformation permanente des cheveux consistant, dans une première étape, à réduire les liaisons disulfures de la kératine par application d'une composition réductrice, puis dans une seconde étape, à reformer lesdites liaisons par application d'une composition oxydante, caractérisé par le fait que l'étape de réduction est réalisée à l'aide d'une composition cosmétique réductrice telle que revendiquée selon l'une quelconque des revendications 1 à 13.

**15.** Procédé selon la revendication 14, caractérisé par le fait que l'on laisse agir la composition réductrice pendant un temps compris entre 5 et 60 min.

**16.** Composés nouveaux caractérisés par le fait qu'ils répondent à la formule générale suivante :

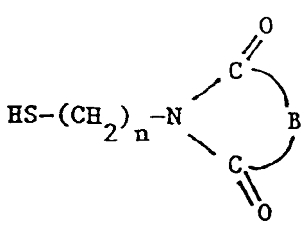

(III)

dans laquelle :
n est 2 ou 3, et
B représente un radical divalent pris dans le groupe constitué par :
(i) $-(CH_2)_3-$
(ii) 1,2-cyclohexylidène, et
(iii) -CH=CH- lorsque dans ce dernier cas n est 3
et leurs disulfures correspondants

**17.** Composés nouveaux caractérisés par le fait qu'ils répondent à la formule générale suivante :

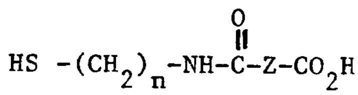 (IV)

dans laquelle :
n est 2 ou 3
(a) lorsque n est 2
Z représente le radical 1,2-cyclohexylidène
(b) lorsque n est 3
Z est un radical divalent choisi dans le groupe constitué par :
(i) $-(CH_2)_m-$, m étant 2 ou 3,
(ii) -CH=CH-
(iii) 1,2-cyclohexylidène, et
(iv) o-phénylène,
et leurs disulfures correspondants, ainsi que le disulfure correpondant lorsque n est 2 et Z représente un radical o-phénylène.

## Patentansprüche

1. Kosmetische Zubereitung zur erstmaligen Erstellung von Dauerwellen, dadurch gekennzeichnet, daß sie in einem entsprechenden kosmetischen Trägermittel als Reduktionsmittel mindestens ein Derivat des N-(Mercaptoalkyl)-bernsteinsäureamids oder des N-(Mercaptoalkyl)-succinimids oder einer ihrer Homologen entsprechend der folgenden allgemeinen Formel enthält:

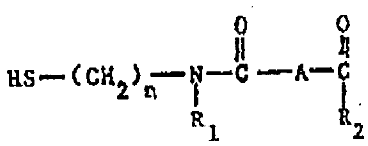 (I)

in der:
n 2 oder 3 ist,
A einen zweiwertigen Rest bedeutet, ausgewählt aus:
(i) $-(CH_2)_m-$, worin m 2 oder 3 ist,
(ii)

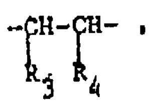 ,

worin $R_3$ und $R_4$, die gleich oder verschieden sind, einen Alkylrest mit 1 bis 4 Kohlenstoffatomen bedeuten, oder $R_3$ und $R_4$ bilden zusammengenommen mit den benachbarten Kohlenstoffatomen einen Cyclohexanring und
(iii)

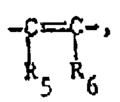 ,

worin $R_5$ und $R_6$, die gleich oder verschieden sind, ein Wasserstoffatom, einen Alkylrest mit 1 bis 4 Kohlenstoffatomen bedeuten, oder $R_5$ und $R_6$ bilden zusammmengenommen mit den benachbarten Kohlenstoffatomen einen Benzolring,
$R_1$ ein Wasserstoffatom bedeutet und
$R_2$ einen Hydroxyrest bedeutet oder
$R_1$ und $R_2$ bilden zusammengenommen eine Einfachbindung, und

die Salze einer organischen oder mineralischen Base der Verbindungen in Form der freien Säure.

2. Zubereitung nach Anspruch 1, dadurch gekennzeichnet, daß es sich bei den Salzen der Verbindungen der Formel (I) in Form der freien Säure um Ammoniumsalze, sekundäre oder tertiäre Aminsalze oder Alkalimetall- oder Erdalkalimetallsalze handelt.

3. Zubereitung nach Anspruch 1 oder 2, dadurch gekennzeichnet, daß, wenn in der allgemeinen Formel (I) $R_1$ ein Wasserstoffatom bedeutet und $R_2$ einen Hydroxyrest darstellt, die Verbindungen ausgewählt sind aus:
N-(2-Mercapto-ethyl)-bernsteinsäureamid,
N-(3-Mercapto-propyl)-bernsteinsäureamid,
N-(2-Mercapto-ethyl)-hexahydrophthalsäureamid,
N-(3-Mercapto-propyl)-hexahydrophthalsäureamid,
N-(2-Mercapto-ethyl)-phthalsäureamid,
N-(3-Mercapto-propyl)-phthalsäureamid,
N-(2-Mercapto-ethyl)-glutarsäureamid,
N-(3-Mercapto-propyl)-glutarsäureamid und
N-(2-Mercapto-ethyl)-maleinsäureamid.

4. Zubereitung nach einem der Ansprüche 1 und 2, dadurch gekennzeichnet, daß, wenn in der allgemeinen Formel (I) $R_1$ und $R_2$ zusammengenommen eine Einfachbindung bilden, die Verbindungen ausgewählt sind aus:
N-(2-Mercapto-ethyl)-succinimid,
N-(3-Mercapto-propyl)-succinimid,
N-(2-Mercapto-ethyl)-glutarimid,
N-(3-Mercapto-propyl)-glutarimid,
N-(2-Mercapto-ethyl)-hexahydrophthalimid und
N-(3-Mercapto-propyl)-hexahydrophthalimid.

5. Zubereitung nach einem der vorhergehenden Ansprüche, dadurch gekennzeichnet, daß die Verbindung der Formel (I) in einer Konzentration zwischen 2 und 20 Gew.-% und vorzugsweise zwischen 5 und 10 Gew.-%, bezogen auf das Gesamtgewicht der Zubereitung, vorhanden ist.

6. Zubereitung nach einem der vorhergehenden Ansprüche, dadurch gekennzeichnet, daß sie einen pH-Wert zwischen 4,5 und 11 und vorzugsweise zwischen 6 und 10 besitzt, den man durch ein alkalisches Mittel ausgewählt aus Ammoniak, Monoethanolamin, Diethanolamin, Triethanolamin, einem alkalischen oder Ammonium-, Mono- oder Bicarbonat, einem Alkalihydroxid oder durch ein Ansäuerungsmittel ausgewählt aus Chlorwasserstoffsäure, Essigsäure, Milchsäure, Oxalsäure oder Borsäure erhält.

7. Zubereitung nach einem der vorhergehenden Ansprüche, dadurch gekennzeichnet, daß sie weiterhin mindestens ein anderes Reduktionsmittel, ausgewählt aus Thioglycolsäure, Monothioglycolat von Glycerin oder Glykol, Cysteamin und seinen $C_1$-$C_4$-Acylderivaten, Cystein, N-Acetyl-Cystein, den Zucker-N-Mercaptoalkylamiden, β-Mercaptopropionsäure und ihren Derivaten, Thiomilchsäure, Thiohydroxybernsteinsäure, Pantethein, Thioglycerin, den Alkalimetall- oder Erdalkalimetallsulfiten und-Bisulfiten, den N-(Mercapto-alkyl)ω-hydroxylamiden, den N-Mono-oder N,N-Dialkylmercapto-4-butyramiden und den Aminomercaptoalkylamiden.

8. Zubereitung nach einem der vorhergehenden Ansprüche, dadurch gekennzeichnet, daß sie weiterhin mindestens ein kationisches Polymer, ein geschmeidig machendes Mittel, ein Proteinhydrolysat, ein Wachs, ein Opacifierungsmittel, ein Parfum, einen Farbstoff, ein kationisches oder nicht-ionisches oberflächenaktives Mittel, ein Behandlungsmittel oder auch ein Penetrierungsmittel enthält.

9. Zubereitung nach einem der vorhergehenden Ansprüche, dadurch gekennzeichnet, daß sie weiterhin mindestens ein Disulfid enthält, wobei die Zubereitung autoneutralisierend ist.

10. Zubereitung nach Anspruch 9, dadurch gekennzeichnet, daß das Disulfid ausgewählt ist aus Dithioglykolsäure, Dithioglycerin, Cystamin, N,N-Diacetylcystamin, Cystin, Pantethin, den Disulfiden von N-(Mercaptoalkyl)ω-hydroxyalkylamiden, den Disulfiden von N-Mono-oder N,N-Dialkylmercapto-4-butyramiden und den Disulfiden von Aminomercaptoalkylamiden.

11. Zubereitung nach Anspruch 9, dadurch gekennzeichnet, daß das Disulfid ein Disulfid ist, die Verbindung der allgemeinen Formel (I) der folgenden allgemeinen Formel entspricht:

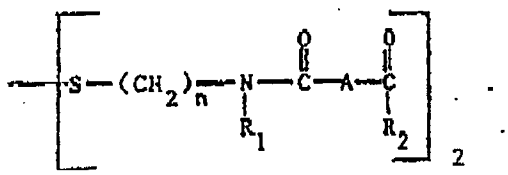 (II)

in der n, $R_1$, $R_2$ und A die in Anspruch 1 gegebenen Bedeutungen aufweisen.

12. Zubereitung nach Anspruch 11, dadurch gekennzeichnet, daß das Disulfid der Formel (II) ausgewählt ist aus:

Bis [N-(3-carboxypropionyl)-N-ethyl]-disulfid
Bis[N-(2-carboxy-cyclohexan-carbonyl)-N-ethyl]-disulfid,
Bis [N-ethylsuccinimid]-disulfid.

13. Zubereitung nach einem der Ansprüche 9 bis 12, dadurch gekennzeichnet, daß das Disulfid in einem molaren Anteil, bezogen auf die Verbindung der Formel (I), von 0,5 bis 2,5 und vorzugsweise von 1 bis 2 vorhanden ist.

14. Verfahren zur Erstellung von permanenten Dauerwellen, bei dem man in einem ersten Schritt die Disulfidbindungen des Keratins durch Aufbringen einer Reduzierungszubereitung reduziert, dann in einem zweiten Schritt die Bindungen durch Aufbringen einer oxidierenden Zusammensetzung wiederherstellt, dadurch gekennzeichnet, daß der Reduzierungsschritt mittels einer kosmetischen Reduzierungszusammensetzung, wie in einem der Ansprüche 1 bis 13 beansprucht, durchgeführt wird.

15. Verfahren nach Anspruch 14, dadurch gekennzeichnet, daß man die Reduzierungszubereitung für einen Zeitraum zwischen 5 und 60 Minuten einwirken läßt.

16. Neue Verbindungen, dadurch gekennzeichnet, daß sie der folgenden allgemeinen Formel entsprechen:

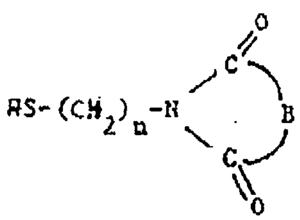 (III)

in der:

n 2 oder 3 ist und
B einen divalenten Rest bedeutet, der aus der Gruppe bestehend aus ausgewählt ist:
(i) $-(CH_2)_3-$,
(ii) 1,2-Cyclohexyliden und
(iii) -CH=CH-, wenn im letzteren Fall n 3 ist,
und die entsprechenden Disulfide.

17. Neue Verbindungen, dadurch gekennzeichnet, daß sie der folgenden allgemeinen Formel entsprechen:

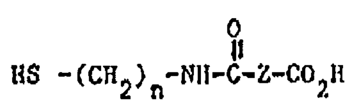 (IV)

in der:

n 2 oder 3 ist,

(a) wenn n 2 ist, ist Z der Rest 1,2-Cyclohexyliden,
(b) wenn n 3 ist, ist Z ein divalenter Rest ausgewählt aus der Gruppe bestehend aus:
(i) $-(CH_2)_m-$, m ist 2 oder 3,
(ii) -CH=CH-,
(iii) 1,2-Cyclohexyliden und
(iv) o-Phenylen,
und die entsprechenden Disulfide ebenso wie das entsprechende Disulfid, wenn n 2 ist und Z einen o-Phenylenrest bedeutet.

## Claims

1. Cosmetic composition for the first stage of an operation for the permanent deformation of the hair, characterised by the fact that it contains, in a cosmetically appropriate vehicle, as reducing agent, at least one N-(mercaptoalkyl)succinamic acid or N-(mercaptoalkyl)succinimide derivative or one of their homologues of the following general formula:

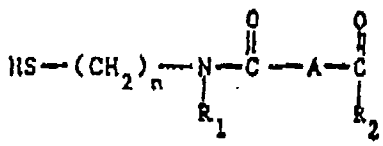

(I)

in which:
n is 2 or 3
A represents a divalent radical chosen from:
(i) $-(CH_2)_m-$, m being 2 or 3,
(ii)

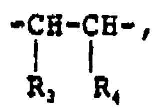

$R_3$ and $R_4$, which are identical or different,
representing an alkyl radical having 1 to 4 carbon atoms or $R_3$ and $R_4$ together form, with the adjacent carbon atoms, a cyclohexane ring, and
(iii)

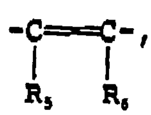

$R_5$ and $R_6$, which are identical or different,

representing a hydrogen atom, an alkyl radical having 1 to 4 carbon atoms or $R_5$ and $R_6$ together form, with the adjacent carbon atoms, a benzene ring,
$R_1$ represents a hydrogen atom and
$R_2$ represents a hydroxyl radical or
$R_1$ and $R_2$ taken together form a single bond,
and the salts with an organic or inorganic base of the said compounds in free acid form.

2. Composition according to Claim 1, characterised by the fact that the salts of the compounds of formula (I) in free acid form are those of ammonium, of a secondary or tertiary amine or of an alkali or alkaline-earth metal.

3. Composition according to Claim 1 or 2, characterised by the fact that when in the general formula (I) $R_1$

represents a hydrogen atom and $R_2$ represents a hydroxyl radical, the compounds are chosen from:

N-(2-mercaptoethyl)succinamic acid,
N-(3-mercaptopropyl)succinamic acid,
N-(2-mercaptoethyl)hexahydrophthalamic acid,
N-(3-mercaptopropyl)hexahydrophthalamic acid,
N-(2-mercaptoethyl)phthalamic acid
N-(3-mercaptopropyl)phthalamic acid,
N-(2-mercaptoethyl)glutaramic acid,
N-(3-mercaptopropyl)glutaramic acid and,
N-(2-mercaptoethyl)maleamic acid.

**4.** Composition according to either of Claims 1 and 2, characterised by the fact that when in the general formula (I), $R_1$ and $R_2$, taken together, form a single bond, the compounds are chosen from:

N-(2-mercaptoethyl)succinimide,
N-(3-mercaptopropyl)succinimide,
N-(2-mercaptoethyl)glutarimide,
N-(3-mercaptopropyl)glutarimide,
N-(2-mercaptoethyl)hexahydrophthalimide and,
N-(3-mercaptopropyl)hexahydrophthalimide.

**5.** Composition according to any one of the preceding claims, characterised by the fact that the compound of formula (I) is present at a concentration of between 2 and 20 % and preferably between 5 and 10 % by weight relative to the total weight of the composition.

**6.** Composition according to any one of the preceding claims, characterised by the fact that it is present at a pH of between 4.5 and 11 and preferably between 6 and 10, obtained by means of an alkaline agent chosen from ammonium hydroxide, monoethanolamine, diethanolamine, triethanolamine, an alkali metal or ammonium carbonate or bicarbonate, an alkali metal hydroxide or by means of an acidifying agent chosen from hydrochloric acid, acetic acid, lactic acid, oxalic acid or boric acid.

**7.** Composition according to any one of the preceding claims, characterised by the fact that it contains, in addition, at least one other reducing agent chosen from thioglycolic acid, glycerol or glycol monothioglycolate, cysteamine and its acylated $C_1$-$C_4$ derivatives, cysteine, N-acetylcysteine, N-mercaptoalkylamides of sugars, β-mercaptopropionic acid and its derivatives, thiolactic acid, thiomalic acid, pantetheine, thioglycerol, sulphites and bisulphites of an alkali or alkaline-earth metal, N-(mercaptoalkyl)-ω-hydroxylamides, N-mono- or N,N-dialkylmercapto-4-butyramides and aminomercaptoalkylamides.

**8.** Composition according to any one of the preceding claims, characterised by the fact that it contains, in addition, at least one cationic polymer, emollient agent, protein hydrolysate, wax, opacifying agent, perfume, colorant, nonionic or cationic surface-active agent, treatment agent or alternatively penetrating agent.

**9.** Composition according to any one of the preceding claims, characterised by the fact that it contains, in addition, at least one disulphide, the composition being self-neutralising.

**10.** Composition according to Claim 9, characterised by the fact that the disulphide is chosen from dithioglycolic acid, dithioglycerol, cystamine, N,N-diacetylcystamine, cystine, pantethine, disulphides of N-(mercaptoalkyl)-ω-hydoxyalkylamides, disulphides of N-mono- or N,N-dialkylmercapto-4-butyramides and disulphides of aminomercaptoalkylamides.

**11.** Composition according to Claim 9, characterised by the fact that the disulphide is a disulphide of a compound of general formula (I) which corresponds to the following general formula:

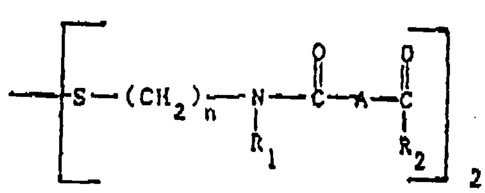

(II)

in which n, $R_1$, $R_2$ and A have the same meanings as those given in Claim 1.

**12.** Composition according to Claim 11, characterised by the fact that the disulphide of formula (II) is chosen from:

bis[N-(3-carboxypropionyl)-N-ethyl]disulphide,
bis[N-(2-carboxycyclohexanecarbonyl)-N-ethyl]disulphide,
bis[N-ethylsuccinimide]disulphide.

**13.** Composition according to any one of Claims 9 to 12, characterised by the fact that the disulphide is present in a mol ratio, relative to the compound of formula (I), ranging from 0.5 to 2.5 and preferably from 1 to 2.

**14.** Process for the permanent deformation of the hair, consisting, in a first stage, in reducing the disulphide bonds of the keratin by application of a reducing composition, then in a second stage, in reforming the said bonds by application of an oxidising composition, characterised by the fact that the reducing stage is performed by means of a reducing cosmetic composition as claimed according to any one of Claims 1 to 13.

**15.** Process according to Claim 14, characterised by the fact that the reducing composition is allowed to act for a period of between 5 and 60 min.

**16.** New compounds characterised by the fact that they correspond to the following general formula:

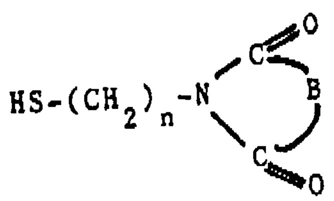

(III)

in which:
n is 2 or 3, and
B represents a divalent radical taken from the group consisting of:
(i) $-(CH_2)_3-$
(ii) 1,2-cyclohexylidene, and
(iii) -CH=CH- when in this last case n is 3, and their corresponding disulphides.

**17.** New compounds characterised by the fact that they correspond to the following general formula:

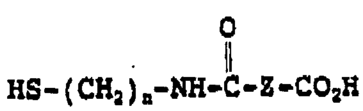

(IV)

in which
n is 2 or 3
(a) when n is 2
Z represents a 1,2-cyclohexylidene radical
(b) when n is 3
Z is a divalent radical chosen from the group consisting of:
(i) $-(CH_2)_m-$, m being 2 or 3,
(ii) -CH=CH-
(iii) 1,2-cyclohexylidene, and
(iv) o-phenylene,
and their corresponding disulphides as well as the corresponding disulphide when n is 2 and Z represents an o-phenylene radical.